**Europäisches Patentamt**

**European Patent Office**

**Office européen des brevets**

(19)

(11) Veröffentlichungsnummer : **0 458 929 B1**

(12) # EUROPÄISCHE PATENTSCHRIFT

(45) Veröffentlichungstag der Patentschrift :
**02.03.94 Patentblatt 94/09**

(51) Int. Cl.$^5$ : **A61K 37/54,** A61K 47/12,
A61K 47/18, A61K 47/22,
A61K 47/20, A61K 47/26

(21) Anmeldenummer : **91900785.6**

(22) Anmeldetag : **19.12.90**

(86) Internationale Anmeldenummer :
**PCT/EP90/02249**

(87) Internationale Veröffentlichungsnummer :
**WO 91/08764 27.06.91 Gazette 91/14**

(54) **t-PA-Solubilisierung.**

(30) Priorität : **20.12.89 DE 3942145**

(43) Veröffentlichungstag der Anmeldung :
**04.12.91 Patentblatt 91/49**

(45) Bekanntmachung des Hinweises auf die
Patenterteilung :
**02.03.94 Patentblatt 94/09**

(84) Benannte Vertragsstaaten :
**AT BE CH DE DK ES FR GB GR IT LI LU NL SE**

(56) Entgegenhaltungen :
**EP-A- 0 211 592**
**EP-A- 0 228 862**
**EP-A- 0 264 887**
**WO-A-90/08557**

(73) Patentinhaber : **BOEHRINGER MANNHEIM
GMBH
D-68298 Mannheim (DE)**

(72) Erfinder : **KOHNERT, Ulrich, Dr.
Heubachweg 6
D-8121 Habach (DE)**
Erfinder : **RAINER, Rudolph, Dr.
Färbergasse 19
D-8120 Weilheim (DE)**

(74) Vertreter : **Huber, Bernhard, Dipl.-Chem. et al
Patentanwälte H. Weickmann, Dr. K. Fincke
F.A. Weickmann, B. Huber Dr. H. Liska, Dr. J.
Prechtel, Dr. B. Böhm Postfach 86 08 20
D-81635 München (DE)**

EP 0 458 929 B1

**Beschreibung**

Der menschliche Gewebe-Plasminogen-Aktivator (t-PA) besitzt eine große therapeutische Bedeutung bei der Auflösung von Blutgerinnseln, z.B. bei Herzinfarkten. t-PA wirkt auf die Blutgerinnungskaskade durch die Aktivierung von Plasminogen zu Plasmin. Plasmin wiederum löst Fibrin, die Hauptkomponente der Proteinmatrix von geronnenem Blut.

Natürlicher t-PA ist aus mehreren funktionellen Domänen F, G, K1, K2 und P zusammengesetzt. Die Domäne P beinhaltet das proteolytisch aktive Zentrum, das die Spaltung von Plasminogen zu Plasmin bewirkt. Die gentechnologische Herstellung von t-PA oder verschiedenen t-PA-Muteinen, bei denen einige der Domänen F, G, K1 und K2 deletiert sind, in eukaryontischen und prokaryontischen Zellen ist bereits bekannt. Dabei werden t-PA-Derivate aus Prokaryonten im Gegensatz zu natürlichem t-PA in nicht glykosylierter Form synthetisiert.

Proteine mit t-PA-Aktivität lösen sich nur in geringer Konzentration in den üblicherweise zur Solubilisierung von Proteinen verwendeten Puffern, wie z.B. 50 mmol/l Na-Citrat, 50 mmol/l Phosphat oder physiologische NaCl-Lösung. Für den Einsatz als therapeutischer Wirkstoff sollten jedoch Proteine mit t-PA-Aktivität mit einer höheren enzymatischen Aktivität von mindestens 0,25 MU/ml, vorzugsweise 0,25 MU/ml bis 10 MU/ml vorliegen.

Aus der EP-A 0 217 379 ist bekannt, die Löslichkeit von t-PA durch neutrale oder leicht alkalische Arginin-Formulierungen zu erhöhen. Ein Nachteil dieses Verfahrens ist jedoch die geringe Stabilität von hochkonzentriertem t-PA unter neutralen oder leicht alkalischen Bedingungen.

Das US-Patent 4,777,043 offenbart eine pharmazeutische Zusammensetzung mit menschlichem t-PA und einem pharmazeutisch verträglichen Argininiumionen enthaltenden Puffer mit einer Chloridionen-Konzentration bis 0,3 mol/l. Die EP-A 0 156 169, die EP-A 0 303 351 und die EP-A 0 297 294 offenbaren weitere Möglichkeiten, Proteine mit t-PA-Aktivität in Puffern durch Aminosäuren, deren Salzen, Derivaten und Homologen zu solubilisieren. Weiterhin kann t-PA durch Zusatz von Gelatine gemäß EP-A 0 123 304, durch Zusatz von Albumin gemäß EP-A 0 112 940 oder durch Zugabe eines Polyschwefelsäureesters eines Saccharids oder eines sulfatierten Zuckers gemäß EP-A 0 198 321 stabilisiert werden. Die PCT/US88/04402 offenbart ein Verfahren zur Erhöhung der t-PA-Löslichkeit, worin man ein wäßriges Medium mit einer basischen Aminosäure, insbesondere Arginin, in einer Konzentration von 0,02 bis 0,2 mol/l zusammen mit einer Citronensäuregruppe in einer Konzentration von 0,02 bis 0,08 mol/l bei einem pH-Wert von 5 bis 8 verwendet.

EP-A-0 211 592 offenbart eine sterile, pharmazeutische Dosierungseinheit von t-PA zur parenteralen Verabreichung, die 0,1 - 15 mg/ml t-PA in einer pharmazeutisch akzeptablen gepufferten wässrigen Lösung bei pH 3,5 - 5,5 enthält. In diesem Dokument werden Präparate mit einer Konzentration von 0,5 mg/ml glykosyliertem t-PA in 100 mmol/l Zitronensäure/Natriumcitrat, pH 5,5, beschrieben. In EP-A-0 211 592 werden jedoch keine t-PA-Muteine oder nicht-glykosylierter t-PA offenbart.

Diese verschiedenen Zusammensetzungen sind jedoch nicht generell für alle Proteine mit t-PA-Eigenschaften geeignet. Insbesondere wurde festgestellt, daß nicht glykosylierter t-PA, nicht glykosylierte t-PA-Muteine und glykosylierter t-PA stark voneinander abweichende Lösungseigenschaften besitzen.

Aufgabe der Erfindung war es demnach, pharmazeutische Präparate zu entwickeln, die glykosylierten und nicht glykosylierten t-PA bzw. t-PA-Muteine mit einer Aktivität von mehr als 0,25 MU/ml enthalten, wobei der t-PA über einen längeren Zeitraum hinweg stabil sein soll.

Die erfindungsgemäße Aufgabe wird gelöst durch ein pharmazeutisches Präparat eines Proteins mit t-PA-Aktivität in einer Konzentration von mindestens 0,4 MU/ml mit einem pH-Wert von 4,5 bis 9, wobei diese Zusammensetzung eine Substanz aus der Gruppe Citronensäure, Ascorbinsäure, 2-Oxoglutarsäure, Fumarsäure, Tris und EDTA in einer Konzentration von mindestens 0,2 mol/l enthält.

Bevorzugt ist eine Konzentration der obengenannten Stoffe von 0,2 bis 1 mol/l, besonders bevorzugt 0,3 bis 1 mol/l.

Geeignet für eine erfindungsgemäße Zusammensetzung ist ein pH-Wert zwischen 4,5 und 9, besonders bevorzugt ist ein pH-Wert von 6 bis 7,5.

Unter Protein mit t-PA-Aktivität gemäß vorliegender Erfindung sind nicht modifizierter t-PA aus prokaryontischen und eukaryontischen Organismen, sowie alle t-PA-Muteine zu verstehen. Beispiele für t-PA-Muteine sind z.B. bei Harris (Protein Engineering 1 (1987), 449-458) beschrieben.

Vorzugsweise enthält die erfindungsgemäße Zusammensetzung nativen glykosylierten t-PA, z.B. aus CHO-Zellen. Enthält ein erfindungsgemäßes Präparat einen nativen glykosylierten t-PA, dann soll seine enzymatische Aktivität vorzugsweise mindestens 1,4 MU/ml betragen.

Die Einheit U ist eine Einheit der Aktivität für t-PA nach Definition der WHO, National Institute for Biological Standards and Control.

Weiterhin bevorzugt ist ein nicht glykosylierter t-PA aus Prokaryonten (t-PA pro), der nach DE-35 37 708 erhältlich ist. Unter t-PA pro versteht man einen t-PA, der mit den Aminosäuren -3 (Gly) bis +1 (Ser) beginnt und bei 527 (Pro) endet (Nomenklatur nach Harris, Protein Engineering, Band 1 (1987) 449-458). Enthält ein erfindungsgemäßes Präparat t-PA pro, dann soll seine enzymatische Aktivität mindestens 0,25 MU/ml betragen.

Weiterhin bevorzugt ist auch ein nicht glykosyliertes t-PA-Mutein mit den Domänen K1, K2 und P, das als K1K2P pro bezeichnet wird, (Herstellung nach DE 39 233 391.1). K1K2P pro beginnt bei Aminosäure 85-92 und endet bei 527 (Pro). Enthält ein erfindungsgemäßes Präparat K1K2P pro, dann soll seine enzymatische Aktivität vorzugsweise mindestens 0,4 MU/ml betragen.

Weiterhin bevorzugt ist auch ein nicht glykosyliertes t-PA-Mutein mit den Domänen K2 und P, das als K2P pro bezeichnet wird (Herstellung nach EP-A 0 382 174). K2P pro beginnt bei Aminosäure 174-179 und endet mit 527 (Pro). Gegebenenfalls kann K2P pro wie auch K1K2P pro teilweise oder ganz die Aminosäuren -3 (Gly) bis +5 (Ile) nach Harris, supra enthalten. Enthält ein erfindungsgemäßes Präparat K2P pro, dann soll seine enzymatische Aktivität vorzugsweise mindestens 1,4 MU/ml betragen. Geeignet sind jedoch auch alle anderen t-PA-Varianten aus Prokaryonten oder Eukaryonten.

Im folgenden ist eine Reihe besonders bevorzugter Präparate gemäß vorliegender Erfindung aufgeführt.

Eine Formulierung enthält 300 mmol/l Citronensäure/NaOH, pH 6. Eine weitere Formulierung enthält 300 mmol/l Ascorbinsäure, pH 6. Wiederum eine weitere Formulierung enthält 300 mmol/l 2-Oxoglutarsäure, pH 6. Wiederum eine weitere Formulierung enthält 300 mmol/l EDTA, pH 6. Wiederum eine weitere Formulierung enthält 300 mmol/l Fumarsäure/NaOH, pH 6. Wiederum eine weitere Formulierung enthält 1 mol/l Tris/HCl, pH 7,2.

Ein Gegenstand der Erfindung ist schließlich auch ein Arzneimittel auf Basis eines Proteins mit t-PA-Aktivität als Wirkstoff in Lösung oder als Lyophilisat mit den angegebenen Wirkstoffen und gegebenenfalls noch weiteren pharmazeutisch verträglichen Zusatz-, Hilfs-, Träger- und Füllstoffen.

Die erfindungsgemäßen pharmazeutischen Präparate kommen vorzugsweise als Injektions- und Infusionslösungen zum Einsatz. Dies kann dadurch geschehen, daß eine bereits spritzfertige Lösung zur Verfügung gestellt wird, die die erfindungsgemäße Zusammensetzung besitzt. Es ist jedoch auch möglich, die pharmazeutischen Präparate in Form von Lyophilisaten zur Verfügung zu stellen. Diese werden dann mit an sich bekannten, zu Injektionszwecken geeigneten Mitteln oder Lösungen rekonstituiert. Als Injektionsmedium kommt vorzugsweise Wasser zur Anwendung, welches die bei Injektionslösungen üblichen Zusätze wie Stabilisierungsmittel, Lösungsvermittler, Puffer und isotonische Zusätze, beispielsweise eine physiologische NaCl-Konzentration, enthält. Derartige Zusätze sind beispielsweise Mannit, Tartrat- oder Citrat-Puffer, Ethanol, Komplexbildner wie z.B. Ethylendiamintetraessigsäure und deren nicht-toxischen Salze, sowie hochmolekulare Polymere, wie flüssiges Polyethylenoxid zur Viskositätsregulierung. Flüssige Trägerstoffe für Injektionslösungen müssen steril sein und werden vorzugsweise in Ampullen abgefüllt.

Schließlich beinhaltet die vorliegende Erfindung auch die Verwendung von glykosylierten Proteinen mit t-PA-Aktivität zur Herstellung erfindungsgemäßer pharmazeutischer Präparaten.

Die folgenden Beispiele sollen die konkrete Ausführung der Erfindung weiter erläutern.

**Beispiel 1**

Einfluß von verschiedenen Substanzen auf die Löslichkeit eines nicht glykosylierten t-PA-Muteins mit der Domänen-Zusammensetzung K2P

In diesem Beispiel wird die Löslichkeit von K2P pro (Herstellung nach EP-A 0 382 174) in verschiedenen Pufferlösungen beschrieben. Wie aus Tabelle 1 zu entnehmen ist, löst sich K2P pro in den aufgeführten Pufferlösungen mit einer deutlich höheren Aktivität als 1,4 MU/ml.

Durchführung

170 ml gereinigtes K2P pro (gelöst in 0,5 mol/l Arginin/Phosphorsäure pH 7,2) werden durch Ultrafiltration über eine Amicon YM 10-Membran konzentriert. Jeweils 1 ml des Konzentrats wird gegen die in Tabelle 1 aufgeführten Puffer dialysiert. Nach Zentrifugation der Proben wird im klaren Überstand die enzymatische Aktivität gemessen.

Die enzymatische Aktivität ist als Volumenaktivität in MU/ml und als Gesamtaktivität in MU angegeben.

Die Messung der t-PA-Aktivität kann dabei auf übliche Weise durch Spaltung eines chromogenen Substrats bestimmt werden (H. Lill, ZGIMAL 42, (1987), 478-486). Die Einheit U ist eine Einheit der Aktivität für t-PA nach Definition der WHO, National Institute for Biological Standards and Control.

## T a b e l l e   1

| Puffer | Aktivität | |
|---|---|---|
| | MU/ml | MU |
| 0,3 mol/l Citronensäure/NaOH, pH 6 | 2,23 | 2,05 |
| 0,3 mol/l Ascorbinsäure/NaOH, pH 6 | 1,68 | 1,95 |
| 1 mol/l Tris/HCl, pH 7,2 | 8,76 | 5,8 |
| 0,3 mol/l EDTA/NaOH, pH 6 | 1,95 | 1,95 |
| 0,3 mol/l 2-Oxoglutarsäure, pH 6 | 2,64 | 3,17 |
| 0,3 mol/l Fumarsäure/NaOH, pH 6 | 1,85 | 4,08 |

**Beispiel 2**

**Löslichkeit von K1K2P pro**

Gereinigtes K1K2P pro (gelöst in 0,5 mol/l Arginin/$H_3PO_4$) wird durch Ultrafiltration über eine YM 10-Membran (Amicon) konzentriert. Jeweils 0,5 ml des Konzentrats (Aktivität: 3,5 MU/ml) wird gegen die in Tabelle 2 aufgeführten Puffer dialysiert. Nach Zentrifugation der Proben wird im klaren Überstand die enzymatische Aktivität gemessen.

Die enzymatische Aktivität ist als Volumeneinheit in MU/ml und als Gesamtaktivität in MU angegeben.

Die Messung der tPA-Aktivität kann dabei auf übliche Weise durch Spaltung eines chromogenen Substrats bestimmt werden (H. Lill, ZGIMAL 42 (1987), 478 - 486). Die Einheit U ist eine Einheit der Aktivität nach Definition der WHO, National Institute for Biological Standards and Control.

## T a b e l l e   2

| Puffer | Aktivität | |
|---|---|---|
| | MU/ml | MU |
| 1 mol/l Tris/HCl, pH 7,2 | 1,65 | 1,07 |

**Beispiel 3**

**Löslichkeit von tPA pro**

Gereinigtes tPA pro (gelöst in 0,5 mol/l Arginin/$H_3PO_4$) wird durch Ultrafiltration über eine YM 10-Membran (Amicon) konzentriert. Jeweils 1 ml des Konzentrats (Aktivität: 2,4 MU/ml) wird gegen die in Tabelle 3 aufgeführten Puffer dialysiert. Nach Zentrifugation der Proben wird im klaren Überstand die enzymatische Aktivität gemessen.

Die enzymatische Aktivität ist als Volumeneinheit in MU/ml und als Gesamtaktivität in MU angegeben.

Die Messung der tPA-Aktivität kann dabei auf übliche Weise durch Spaltung eines chromogenen Substrats bestimmt werden (H. Lill, ZGIMAL 42 (1987), 478 - 486). Die Einheit U ist eine Einheit der Aktivität nach Definition der WHO, National Institute for Biological Standards and Control.

## Tabelle 3

| Puffer | Aktivität | |
|---|---|---|
| | MU/ml | MU |
| 0,3 M Citronensäure/NaOH, pH 6 | 0,29 | 0,16 |
| 1 M Tris/HCl, pH 7,2 | 2,15 | 1,07 |

### Beispiel 4

### Löslichkeit von CHO-tPA

Gereinigtes CHO-tPA (gelöst in 0,5 mol/l Arginin/$H_3PO_4$) wird durch Ultrafiltration über eine YM 10-Membran (Amicon) konzentriert. Jeweils 1 ml des Konzentrats (Aktivität: 6,6 MU/ml) wird gegen die in Tabelle 4 aufgeführten Puffer dialysiert. Nach Zentrifugation der Proben wird im klaren Überstand die enzymatische Aktivität gemessen.

Die enzymatische Aktivität ist als Volumeneinheit in MU/ml und als Gesamtaktivität in MU angegeben.

Die Messung der tPA-Aktivität kann dabei auf übliche Weise durch Spaltung eines chromogenen Substrats bestimmt werden (H. Lill, ZGIMAL 42, (1987), 478-486). Die Einheit U ist eine Einheit der Aktivität nach Definition der WHO, National Institute for Biological Standards and Control.

## Tabelle 4

| Puffer | Aktivität | |
|---|---|---|
| | MU/ml | MU |
| 1 M Tris/HCl, pH 7,2 | 6,16 | 6,49 |
| 0,3 M Citronensäure/NaOH pH 6 | 4,44 | 4,44 |

### Patentansprüche

1.  Pharmazeutisches Präparat eines Proteins mit t-PA- Aktivität mit einer enzymatischen Aktivität von mindestens 0,25 MU/ml und einem pH-Wert von 4,5 bis 9, **dadurch gekennzeichnet**, daß es eine Substanz aus der Gruppe Citronensäure, Ascorbinsäure, 2-Oxoglutarsäure, Fumarsäure, Tris und EDTA in einer Konzentration von mindestens 0,2 mol/l enthält.

2.  Pharmazeutisches Präparat nach Anspruch 1, **dadurch gekennzeichnet**, daß die Konzentration der obengenannten Substanzen 0,2 bis 1 mol/l beträgt.

3. Pharmazeutisches Präparat nach Anspruch 1, **dadurch gekennzeichnet**, daß die Konzentration der obengenannten Substanzen 0,3 bis 1 mol/l beträgt.

4. Pharmazeutisches Präparat nach einem der Ansprüche 1 bis 3, **dadurch gekennzeichnet**, daß das Protein mit t-PA-Aktivität die Domänenzusammensetzung K2P besitzt, nicht glykosyliert ist und eine enzymatische Aktivität von mindestens 1,4 MU/ml besitzt.

5. Pharmazeutisches Präparat nach einem der Ansprüche 1 bis 3, **dadurch gekennzeichnet**, daß das Protein mit t-PA-Aktivität die Domänenzusammensetzung K1K2P besitzt, nicht glykosyliert ist und eine enzymatische Aktivität von mindestens 0,4 MU/ml besitzt.

6. Pharmazeutisches Präparat nach einem der Ansprüche 1 bis 3, **dadurch gekennzeichnet**, daß das Protein mit t-PA-Aktivität ein nicht glykosylierter t-PA pro ist und eine enzymatische Aktivität von mindestens 0,25 MU/ml besitzt.

7. Pharmazeutisches Präparat nach einem der Ansprüche 1 bis 3, **dadurch gekennzeichnet**, daß das Protein mit t-PA-Aktivität ein glykosylierter t-PA mit einer enzymatischen Aktivität von mindestens 1,4 MU/ml ist.

8. Pharmazeutisches Präparat nach einem der vorhergehenden Ansprüche, **dadurch gekennzeichnet**, daß es 300 mmol/l Citronensäure/NaOH, pH 6 enthält.

9. Pharmazeutisches Präparat nach einem der vorhergehenden Ansprüche, **dadurch gekennzeichnet**, daß es 300 mmol/l Ascorbinsäure/NaOH, pH 6 enthält.

10. Pharmazeutisches Präparat nach einem der vorhergehenden Ansprüche, **dadurch gekennzeichnet**, daß es 300 mmol/l 2-Oxoglutarsäure/NaOH, pH 6 enthält.

11. Pharmazeutisches Präparat nach einem der vorhergehenden Ansprüche, **dadurch gekennzeichnet**, daß es 300 mmol/l EDTA/NaOH, pH 6 enthält.

12. Pharmazeutisches Präparat nach einem der vorhergehenden Ansprüche, **dadurch gekennzeichnet**, daß es 300 mmol/l Fumarsäure/NaOH, pH 6 enthält.

13. Pharmazeutisches Präparat nach einem der vorhergehenden Ansprüche, **dadurch gekennzeichnet**, daß es 1 mol/l Tris/HCl, pH 7,2 enthält.

14. Pharmazeutisches Präparat auf Basis eines Proteins mit t-PA-Aktivität als Wirkstoff, **gekennzeichnet durch** eine Zusammensetzung nach einem der vorhergehenden Ansprüche, gegebenenfalls zusammen mit üblichen pharmazeutischen Zusatz-, Hilfs- oder/und Trägerstoffen.

15. Verfahren zur Herstellung eines pharmazeutischen Präparats nach einem der Ansprüche 1 bis 14, **dadurch gekennzeichnet**, daß man ein Protein mit t-PA-Aktivität zusammen mit einer Substanz aus der Gruppe nach Anspruch 1 in eine geeignete pharmazeutische Darreichungsform überführt.

16. Verfahren nach Anspruch 15, **dadurch gekennzeichnet**, daß die pharmazeutische Darreichungsform eine Injektionslösung oder ein Lyophilisat ist.

17. Verwendung eines Proteins mit t-PA-Aktivität zur Herstellung von pharmazeutischen Präparaten nach einem der Ansprüche 1 bis 14.

## Claims

1. Pharmaceutical preparation of a protein with t-PA activity with an enzymatic activity of at least 0,25 MU/ml and 8 pH value of 4.5 to 9, characterised in that it contains a substance from the group citric acid, ascorbic acid, 2-oxoglutaric acid, fumaric acid, Tris and EDTA in a concentration of at least 0.2 mol/l.

2. Pharmaceutical preparation according to claim 1, characterised in that the concentration of the above-mentioned substances amounts to 0.2 to 1 mol/l.

3. Pharmaceutical preparation according to claim 1, characterised in that the concentration of the above-mentioned substances amounts to 0.3 to 1 mol/l.

4. Pharmaceutical preparation according to one of claims 1 to 3, characterised in that the protein with t-PA activity possesses the domain composition K2P is not glycosylated and possesses an enzymatic activity of at least 1.4 MU/ml.

5. Pharmaceutical preparation according to one of claims 1 to 3, characterised in that the protein with t-PA activity possesses the domain composition K1K2P, is not glycosylated and possesses an enzymatic activity of at least 0.4 MU/ml.

6. Pharmaceutical preparation according to one of claims 1 to 3, characterised in that the protein with t-PA activity is a non-glycosylated t-PA pro and possesses an enzymatic activity of at least 0.25 MU/ml.

7. Pharmaceutical preparation according to one of claims 1 to 3, characterised in that the protein with t-PA activity is glycosylated t-PA with an enzymatic activity of at least 1.4 MU/ml.

8. Pharmaceutical preparation according to one of the preceding claims, characterised in that it contains 300 mmol/l citric acid/NaOH, pH 6.

9. Pharmaceutical preparation according to one of the preceding claims, characterised in that it contains 300 mmol/l ascorbic acid/NaOH, pH 6.

10. Pharmaceutical preparation according to one of the preceding claims, characterised in that it contains 300 mmol/l 2-oxoglutaric acid/NaOH, pH 6.

11. Pharmaceutical preparation according to one of the preceding claims, characterised in that it contains 300 mmol/l EDTA/NaOH, pH 6.

12. Pharmaceutical preparation according to one of the preceding claims, characterised in that it contains 300 mmol/l fumaric acid/NaOH, pH 6.

13. Pharmaceutical preparation according to one of the preceding claims, characterised in that it contains 1 mol/l Tris/HCl, pH 7.2.

14. Pharmaceutical preparation based on a protein with t-PA activity as active material, characterised by a composition according to one of the preceding claims, possibly together with usual pharmaceutical additive, adjuvant and/or carrier materials.

15. Process for the production of a pharmaceutical preparation according to one of claims 1 to 14, characterised in that one converts a protein with t-PA activity, together with a substance from the group according to claim 1, into a suitable pharmaceutical form of administration.

16. Process according to claim 15, characterised in that the pharmaceutical form of administration is an injection solution or a lyophilisate.

17. Use of a protein with t-PA activity for the production of pharmaceutical preparations according to one of claims 1 to 14.

## Revendications

1. Préparation pharmaceutique d'une protéine présentant l'activité du t-PA et ayant une activité enzymatique d'au moins 0,25 MU/ml et un pH de 4,5 à 9, caractérisée en ce qu'elle contient une substance du groupe de l'acide citrique, de l'acide ascorbique, de l'acide 2-oxoglutarique, de l'acide fumarique, du Tris et de l'EDTA en une concentration d'au moins 0,2 mol/l.

2. Préparation pharmaceutique selon la revendication 1, caractérisée en ce que la concentration des substances citées ci-dessus est de 0,2 à 1 mol/l.

3. Préparation pharmaceutique selon la revendication 1, caractérisée en ce que la concentration des substances citées ci-dessus est de 0,3 à 1 mol/l.

4. Préparation pharmaceutique selon l'une des revendications 1 à 3, caractérisée en ce que la protéine présentant l'activité du t-PA possède la composition de domaines K2P, n'est pas glycosylée et possède une activité enzymatique d'au moins 1,4 MU/ml.

5. Préparation pharmaceutique selon l'une des revendications 1 à 3, caractérisée en ce que la protéine présentant l'activité du t-PA possède la composition de domaines K1K2P, n'est pas glycosylée et possède une activité enzymatique d'au moins 0,4 MU/ml.

6. Préparation pharmaceutique selon l'une des revendications 1 à 3, caractérisée en ce que la protéine présentant l'activité du t-PA est un t-PA pro non glycosylé et possède une activité enzymatique d'au moins 0,25 MU/ml.

7. Préparation pharmaceutique selon l'une des revendications 1 à 3, caractérisée en ce que la protéine présentant l'activité du t-PA est un t-PA glycosylé possédant une activité enzymatique d'au moins 1,4 MU/ml.

8. Préparation pharmaceutique selon l'une des revendications précédentes, caractérisée en ce qu'elle contient 300 mmol/l d'acide citrique/NaOH, pH 6.

9. Préparation pharmaceutique selon l'une des revendications précédentes, caractérisée en ce qu'elle contient 300 mmol/l d'acide ascorbique/NaOH, pH 6.

10. Préparation pharmaceutique selon l'une des revendications précédentes, caractérisée en ce qu'elle contient 300 mmol/l d'acide 2-oxoglutarique/NaOH, pH 6.

11. Préparation pharmaceutique selon l'une des revendications précédentes, caractérisée en ce qu'elle contient 300 mmol/l d'EDTA/NaOH, pH 6.

12. Préparation pharmaceutique selon l'une des revendications précédentes, caractérisée en ce qu'elle contient 300 mmol/l d'acide fumarique/NaOH, pH 6.

13. Préparation pharmaceutique selon l'une des revendications précédentes, caractérisée en ce qu'elle contient 1 mol/l de Tris/HCl, pH 7,2.

14. Préparation pharmaceutique à base d'une protéine présentant l'activité du t-PA en tant que principe actif, caractérisée par une composition selon l'une des revendications précédentes, éventuellement avec des additifs, adjuvants et/ou véhicules pharmaceutiques habituels.

15. Procédé d'obtention d'une préparation pharmaceutique selon l'une des revendications 1 à 14, caractérisé en ce que l'on convertit en une forme d'administration pharmaceutique appropriée une protéine présentant l'activité du t-PA conjointement avec une substance du groupe selon la revendication 1.

16. Procédé selon la revendication 15, caractérisé en ce que la forme d'administration pharmaceutique est une solution pour injection ou un lyophilisat.

17. Utilisation d'une protéine présentant l'activité du t-PA pour l'obtention de préparations pharmaceutiques selon l'une des revendications 1 à 14.